# EUROPEAN PATENT SPECIFICATION

(11) **EP 2 196 211 B1**
(45) Date of publication and mention of the grant of the patent: **02.04.2014**
(21) Application number: 08741791.1
(22) Date of filing: 27.02.2008
(51) Int. Cl.: A61K 38/21, A61K 38/40, A61P 31/12

(54) **APOLACTOFERRIN COMPOSITIONS FOR USE IN TREATING VIRAL HEPATITIS C**
APOLACTOFERRIN-ZUSAMMENSETZUNGEN ZUR VERWENDUNG IN DER BEHANDLUNG VON VIRALER HEPATITIS C
COMPOSITIONS À BASE D'APOPROTÉINE DE LACTOFERRINE POUR L'UTILISATION DANS LE TRAITEMENT DE L'HÉPATITE C VIRALE

(30) Priority: 05.09.2007 US 970159 P
(43) Date of publication of application: 16.06.2010
(73) Proprietor: Shatunovsky, Nikolai Evgenievich, Moscow 125319 (RU)
(72) Inventor: Shatunovsky, Nikolai Evgenievich, Moscow 125319 (RU)
(74) Representative: HOFFMANN EITLE
(86) International application number: PCT/RU2008/000109
(87) International publication number: WO 2009/031918

(56) References cited:
- EP-A1- 0 659 436
- EP-A1- 1 116 490
- EP-A1- 1 352 657
- WO-A1-2005/016370
- "EFFECT OF LACTOFERRIN FOR PATIENTS WITH CHRONIC HEPATITIS C", HEPATOLOGY, WILLIAMS AND WILKINS, BALTIMORE, MD, US, 1 October 2000 (2000-10-01), page 444A, XP002909013, ISSN: 0270-9139, DOI: DOI:10.1053/JHEP.2000.9873
- AZZAM H S ET AL: "Natural products and chronic hepatitis C virus", LIVER INTERNATIONAL, BLACKWELL MUNKSGAARD, OXFORD, GB, vol. 27, no. 1, 1 February 2007 (2007-02-01), pages 17-25, XP009113444, ISSN: 1478-3223, DOI: DOI:10.1111/J.1478-3231.2006.01408.X [retrieved on 2007-01-16]
- BAKER EN ET AL.: 'Molecular structure,bindmg properties and dynamics of lactoferrin' CELL MOL.LIFE SCI. vol. 62, no. 22, November 2005, pages 2531 - 2539, XP019200886
- ISHIBASHI Y.ET AL.: 'Randomized placebo-controlled trial of interferon aplha-2b plus riba-virin with and without lactoferrim for chronic hepatitis C' HEPATOL.RES. vol. 32, no. 4, August 2005, pages 218 - 223, XP005042311

## Description

### Field of invention.

The present invention relates to medicine, particularly to hepatology and virology, and relates to the use of lactoferrin in the treatment of chronic infection, caused by hepatitis C virus (HCV). More particularly, this invention provides pharmaceutical compositions, comprising apolactoferrin and interferon, and methods of using these compositions in the treatment of chronic infection, caused by hepatitis C virus.

### Background of invention.

HCV belongs to the family of RNA-containing viruses, Flaviviridae, and causes infectious processes in people with hepatitis being the most frequent complication, often developing into hepatic cirrhosis and hepatic carcinoma (Surveillance. Hepatitis. CDC Report Nº61; Younossi Z, Kallman J, Kincaid J. The effects of HCV infection and management on health-related quality of life. Hepatology. 2007 Mar;45(3):806-16). HCV infection has affected over 170 million people globally and the number of the infected continues to increase. In the entire world there are about 1.5 million of cases of HCV-related hepatic carcinoma. Losses, attributed to HCV infection in the United States alone are estimated to be 200 million (USD) annually.

In the total majority of cases the people, infected with HCV, do not experience any severe health problems or demonstrate very mild symptoms. However, when the infection develops for a long time, the occurrence of hepatitis is frequent, being characterized by pain and hepatic dysfunction. Anti-HCV antibodies appear in the blood of the people who have been infected, and after some time the viral RNA appears as well. In the majority of cases the viral RNA is not detected in blood.

HCV possesses both a powerful ability to suppress the human immune system and an ability to get round it due to "adaptive" mutations. Currently there are no commercially available vaccines against HCV infection.

Methods of treating HCV have been and still are based on using interferon, administered in megadosage, in particular, genetic engineering interferon preparations (Shepherd J, Jones J, Hartwell D, Davidson P, Price A, Waugh N. Interferon alfa (pegylated and non-pegylated) and ribavirin for the treatment of mild chronic hepatitis C: a systematic review and economic evaluation. Health Technol Assess. 2007 Mar;11(11):1-224; Younossi Z, Kallman J, Kincaid. J. The effects of HCV infection and management on health-related quality of life. Hepatology. 2007 Mar;45(3):806-16). However, the approaches to treatment, based on genetic engineering interferons, have a relatively low efficiency that ranges between 8 and 25%, according to different sources. The duration of this treatment can reach several years and the recurring frequency comprises 70-80%. Besides, the usage of genetic engineering interferons has severe side effects that include dyshematopoiesis, gastroenteric upsets, hair loss, depression and insomnia.

The wide-spread modem tendency of treating HCV is using complex therapy that adds to genetic engineering interferons a cocktail, containing both general antivirals and one or two inhibitors of HCV propagation (specific polymerase-helicase and/or RNA-polymerase inhibitors) (Toniutto P, Fabris C, Bitetto D, Fornasiere E, Rapetti R, Pirisi M. Valopicitabine dihydrochloride:a specific polymerase inhibitor of hepatitis C virus. Curr Opin Investig Drugs. 2007 Feb;8(2):150-8; Johnson CL, Owen DM, Gale M Jr. Functional and therapeutic analysis of hepatitis C virus NS3.4A protease control of antiviral immune defense. J Biol Chem. 2007 Apr;282(14):10792-803). Such cocktails increase the recovery proportion, however, they inevitably lead to selection of HCV adaptive mutants, resistant to these inhibitors. These clinical problems have vividly manifested themselves in the process of conquering the human immunodeficiency virus and, evidently, will be at least as actual as that for HCV.

Due to shortcomings of the therapy, based on recombinant interferons and inhibitors of viral enzymes, searching for preparations that will fight HCV effectively has been and still is an important task. Development of new combined methods of therapy based on interferons, combined with other antivirals, seems promising for this purpose.

Lactoferrin is a natural iron-binding protein that is present in lacrimal liquid, saliva, peripheral blood, particularly in leucocytes, breast milk, colostrum, glandular epithelium secretion, etc. Lactoferrin is capable of binding two Fe atoms per a molecule. Currently there is no common opinion about value of lactoferrin molecular weight in scientific and patent literature. Thus, lactoferrin considered to have a molecular weight about 80000 Da (see, for example, EP 0 253 395 B2). At the same time in literature there are such molecular weight values for bovine lactoferrin, as 76000 Da (Farrell H.M., Jr., et al. Nomenclature of Proteins of Cows' Milk - Sixth Revision. J. Dairy Sci. (2004), v.87, p. 1641-1674), 86000 Da (Kazutomo Imabori and Tamio Yamakawa, Eds., "Dictionary of Biochemistry", 2nd edition, p.1390, Tokyo Kagaku Dojin Co., 1990) and even 93000 Da (Weiner R.E. and Szuchet S. The molecular weight of bovine lactoferrin. Biochim. Biophys. Acta (1975), v. 393(1), p.143-147). For protein from human breast milk provided such molecular weight values, as, for example, 76000 Da (Moguilevsky N., et al. Comparison of human lactoferrins from milk and neutrophilic leucocytes. Biochem. J. (1985), v.229, p.353-359) and 88000 Da (Kazumoto Imabori and Tamio Yamakawa, Eds., "Dictionary of Biochemistry", 2nd edition, p.1390, Tokyo Kagaku Dojin Co., 1990). Probably, the variances in provided molecular weight values caused by using of various analytical methods, as well as different extent and/or profile of protein glycosylation.

Lactoferrin and its derivates possess significant antioxidant, antimicrobial (J. Pediatr., 1979, v. 94, p. 1), immunomodulatory and antineoplastic actions.

There are the methods of treating for viral diseases, in particular, caused by influenza virus, human immunodeficiency virus, hepatitis C virus, and others, with using of lactoferrin or its fragments. Lactoferrin was offered as reinforcing agent for the therapeutic effect of interferon in treating chronic hepatitis C (patent application EP 1 352 657, 15.10.2003). Although this paper contains a wide generalization that allows to think that in order to reinforce the therapeutic effect of interferon and, hence, for successful implementation of the declared method of treating chronic hepatitis C lactoferrin can be used in any form, including lactoferrin, not saturated with metal, lactoferrin, saturated with various metals, and proper apolactoferrin, and that it can be administered orally or injected, this paper presents experimental data, confirming possibility of using natural lactoferrin only, administered orally only. This paper demonstrates that oral administration of large doses of lactoferrin (at least 1 mg per 1 kg of weight daily, in particular, 1.8 g daily for 6 months) together with administering interferon results in positive therapeutic effect. However, the obtained result cannot be considered fully satisfactory since 6 months after the treatment course ended, complete eradication of the virus and normalization of hepatic function were recorded only in 33.3% of the cases and in the remaining 66.7% of the cases either the treatment was ineffective at all or the virus appeared in blood again since 6 months after the treatment course ended, regardless of the fact that hepatic function normalization was observed.

Thus, in this area there is an acute necessity of developing new medications for treating viral hepatitis C as well as pharmaceutical compositions and methods of treatment, based on them, that would combine high efficiency, in particular, ability of complete eradication of the virus and prevention of the disease recurrence, low cost, storage stability and possibility of reaching therapeutic effect sooner than when using existing methods and schemes of treatment.

### Summary of the invention.

This invention has become possible as a result of the inventors' sudden discovery of the fact that administering lactoferrin that essentially does not contain bound Fe ion (apoprotein) is extremely effective for treating viral hepatitis C. The present invention is defined by the claims.

Accordingly, in first aspect the present invention relates to composition comprising human lactoferrin and interferon for use in the treatment of HCV infection, wherein the lactoferrin has an iron content of not exceeding 6% and wherein lactoferrin comprised in said composition has at least 80% amino sequence homology with the sequence of human lactoferrin. In one embodiment, said HCV infection is a chronic HCV infection, in yet another embodiment, said HCV is the genotype 1b HCV.

In yet other embodiment of composition in accordance with present invention the human lactoferrin has the apparent molecular weight about 80 kDa.

In one embodiment of composition the human lactoferrin present in amount of about 20 mg. In yet another embodiment, a composition for use in the treatment of HCV infection by administration to a patient is disclosed, wherein said composition comprised human lactoferrin as defined above, and wherein said treatment further comprises administering interferon to the patient.

In yet one of embodiments of present invention, that patient is chronically infected with HCV. In yet one of embodiments of present invention method HCV belongs to 1b genotype.

In one instance of the present disclosure comprising the present invention, elimination of HCV from patient body for a period of more than 3 months is achieved.

In its next aspect the present invention relates to the kit for use in the treatment of HCV infection, comprising:
a. a first container having therein a therapeutically effective amount of human lactoferrin wherein said lactoferrin has the content of iron of not exceeding 6%,
b. a second container having therein a therapeutically effective amount of interferon.

In other embodiment of kit, the human lactoferrin has an apparent molecular weight of about 80 kDa.

In one of embodiments of the kit according with present invention, the human lactoferrin is present in the composition in amount of about 20 mg.

In other embodiment the kit is intended for the treatment of HCV infection, which is a chronic infection.

In other embodiment the kit is intended for the treatment of HCV infection caused by virus, having 1b genotype.

The declared group of inventions is aimed at responding to a united challenge of creating a low cost preparation, that would possess an antiviral (hepatitis C) action for the human body as a whole.

Clinical usage of the described preparation in pharmaceutical practice and the method of its administration allow to obtain a number of technical, medical and economic results:
1. The preparation is biologically compatible with the human body and it is therapeutically highly effective in treating chronic viral hepatitis C, it can be combined with administering interferons and with chemotherapy.
2. Medications of necessary concentration can be made on the basis of the preparation.
3. The preparation is easily stored and transported.
4. Medications, made on the basis of the preparation, can be administered to a human body intravenously.
5. The preparation is affordable, its usage in medical practice is economically justified.
6. Administering the preparation, combined with interferons and chemotherapy, for treating chronic viral hepatitis C allows to reach a sustainable antiviral effect within a much shorter period of time (10-20 times quicker) than when using the known schemes of treating chronic viral hepatitis C.
7. The preparation considerably enhances efficiency of the existing treatment scheme for chronic viral hepatitis C, reducing the time of complete recovery to 2-3 weeks' course.

### Brief Description of the Drawings

Fig. 1 depicts the results of analyzing the purity of various samples of human lactoferrin by SDS PAGE under denaturing conditions. Path A is a molecular weight marker, Path B is a standard specimen of human lactoferrin, Paths 1-3 are samples of human lactoferrin (commercially available preparation of Laprot), Paths 4-13 are samples of human lactoferrin apoprotein.
Fig. 2 depicts the results of chromatographic analysis of human lactoferrin (commercially available preparation Laprot) by HPLC.
Fig. 3 depicts the results of chromatographic analysis of human lactoferrin by HPLC.

### Definitions

The expression "essentially free of iron" refers to the lactoferrin composition having a degree of iron saturation less than approximately 5%, however, the disclosure also contemplates higher iron saturation degrees, including, but not limited to between 5% and 20%.

The expression "less than about 5%" refers to a lactoferrin composition having iron content not exceeding 6%, such as, for example, 5.9%.

The phrase "present in an amount of about mg/µg" refers to an amount of lactoferrin that is effective in the treatment of HCV. This amount includes, but is not limited to an amount that is up to 15% more or less than mg/µg.

The preparation of this invention is preferably administered, while included in pharmaceutical composition that contains as active ingredient a therapeutically effective amount of lactoferrin substantially in the form of apoprotein with molar weight about 80000 Daltons and pharmaceutically acceptable vehicle, filler or excipient. As used herein, the phrase "about 80000 Daltons" refers to the weight, which is less than or more than 80000 Daltons to up to 15%. "Pharmaceutically acceptable vehicle, filler or excipient" mean such a vehicle, filler or excipient that does not cause any undesirable side effects for the patient that it is administered to. These pharmaceutically acceptable vehicles, fillers or excipients are well-known in this area (see, for example, Remington's Pharmaceutical Sciences, 18th edition, A.R.Gennaro, Ed., Mack Publishing Company, 1990; Pharmaceutical Formulation Development of Peptides and Proteins, S.Frokjaer and L. Hovgaard, Eds., Taylor and Francis, 2000; and Handbook of Pharmaceutical Excipients, 3rd edition, A. Kibbe, Ed., Pharmaceutical Press, 2000).

Examples of pharmaceutically acceptable vehicles, fillers or excipients include (but are not limited to) buffer agents, agents for maintaining isotonicity, preservatives, surface-active substances (ionic and non-ionic), fillers, stabilizers, etc.

Apoprotein of lactoferrin may be included in the pharmaceutical composition of this invention, using well-known techniques. Suitable techniques for including it into a composition are described, for instance, in Remington's Pharmaceutical Sciences, 17th edition (Mack Publ. Co., Philadelphia, PA, 1985).

The pharmaceutical compositions for use in the treatment of HCV infection according to this invention can be administered alone or combined with one or more other therapeutic agents. These agents can be included in the same composition as apolactoferrin, or they may be present in separate compositions, such as, for example, in kit.

In some embodiments the composition for use in the treatment of HCV infection according to the present invention includes a therapeutically effective amount of apolactoferrin in combination with interferon. Interferon suitable for use in the present invention can be in any form that provides a therapeutic effect when injected according, for example, with the methods disclosed in the present invention. In on non-limiting embodiment of present invention apolactoferrin compositions of the present invention can be prepared in conjunction with or may be introduced in combination with interferon alpha-2.

The pharmaceutical composition of this invention can be structured as a number of drug products, e.g. as solution, emulsion, lyophilizated powder for dissolution ex tempore. Specifically, the pharmaceutical composition of apolactoferrin in this invention can present a lyophilizated or stabilized dissolved product. The lyophilizated product can be prepared by using a number of methods, known in this area. This product, as professionals know, provides for stability of the composition for a long time. The advantage of obtaining a liquid stabilized composition is in the simplicity of use and in a quicker action in emergency situations that potentially may save a patient's life. A liquid composition may also be frozen, thus, presupposing its defrosting directly before administering it to a patient.

A specialist in this sphere, e.g. an attending medical doctor, may prescribe an additional medication or non-medication therapy, being guided by the patient's condition, presence of concomitant diseases, clinical feasibility, while taking into account the known information about compatibility of medical preparations.

The apolactoferrin compositions can be administered by any of ways that provide therapeutic effect for HCV infection, including chronic HCV infection. For example, compositions can be administered parenterally, e.g. intravenously, intraarterially, intraperitoneally, intramuscularly or trough any combinations of the above. It is preferable to administer the pharmaceutical composition of this invention intravenously. This composition for intravenous administration can be in the form of solution, suspension, emulsion of the type oil-in-water, water-in-oil, liposome suspension. Such well-known pharmaceutically acceptable solvents as isotonic 0.9% saline solution (normal saline solution), 5% glucose (dextrose) solution, mannite solution, etc. can be used as solvents for preparing the solution for intravenous administration. If necessary, the solution can additionally contain auxiliary components, e.g. stabilizers, surface-active substances, antimicrobial agents (preservatives), etc.

The techniques of obtaining sterile pyrogen-free solutions for parenteral administration are well-known to professionals in this area and presuppose using sterile pyrogen-free water and sterilizing the obtained solution, e.g. by filtering it through a sterilizing filter with pores size of 0.4 microns, for instance.

In one of non-limiting examples of the invention human apolactoferrin is administered intravenously in treatment of chronic HCV. The composition injected intravenously at a dose 20 mg every 2 hours in the first and second day of the therapeutic scheme. From the day 3 to day 7 of scheme apolactoferrin composition injected into the daily dose of 20 mg. From the day 3 to day 7 also administrated hyperdoses of interferon (e.g. interferon alpha-2) under the cover of extracorporal detoxification methods.

Experimental and clinical data point out the existence of limiting doses for human lactoferrin (both single and course doses) when lower doses are not effective for lactoferrin-based medications and higher ones can result in severe adverse reactions. There have been declared single and course doses for the pharmaceutical for intravenous administration, based on the preparation, containing human apolactoferrin, which are expedient for treating chronic viral hepatitis C.

Not intending to be limited by any scientific theories or doctrines, the inventors connect the vivid hepatoprotective effect, observed when using apolactoferrin for treating viral hepatitis C, with the fact that the cells, infected with hepatitis C virus, under the influence of apolactoferrin end their life with apoptosis and not with necrosis and it prevents the emission of more and more new portions of the virus into the systemic blood circulation.

The preferable way of administering lactoferrin apoprotein, that is, intravenous, provides for a quick and purposeful transportation of apolactoferrin in an unaltered condition to the target organ (the liver), thus, guaranteeing maximum accumulation of the active ingredient in the liver.

In the context of this research the term "therapeutically effective quantity" means the quantity of the pharmaceutical that will guarantee the desirable effect, regarding the treated disease. In particular, the therapeutically effective quantity must be capable of preventing and diminishing the severity or spread of the disease that is being treated. The desirable effect may consist in lowering the viral particle titer in the patient's blood or in complete elimination of the virus from the blood circulation, in hepatoprotective function, etc. The most desirable effect of the treatment would be complete eradication of the viral hepatitis C causal organism from the patient's body.

Therapeutically effective quantities may be determined by a specialist in this area individually, while taking into account such variables as the patient's weight and age, general health, severity of the main disease, presence of concomitant diseases, etc. The administration scheme, including the dosage of components for this drug therapy of this invention, the method and the frequency of administration, the course treatment duration may also be determined by a specialist in this area routinely and, if necessary, may be corrected in the course of treatment, taking into account laboratory research data, clinical observation, symptoms dynamics, etc.

Human lactoferrin may be extracted from human colostrums, foremilk or milk and other human biological material, as well as received by genetic engineering or transgenic methods. Lactoferrin may be extracted from human milk serum by way of desalting and cation exchange chromatography, as it is described in the USSR patent No. 1709606 of March 01, 1990. There is also a known method of extracting lactoferrin from human milk by way of diafiltration-ultrafiltration (international publication of application WO 89/11226, November 30, 1989). There was described a method of one-stage lactoferrin purification from human milk by way of affinity chromatography on a column with immobilized monoclonal antibody against human lactoferrin (patent JP60100019, August 29, 1985). All the enumerated methods as well as other methods may be successfully used for getting native lactoferrin (in the form of holoprotein, containing bound iron).

Expression of gene, encoding human lactoferrin, observed not only in the mammary gland, but also in other tissue. For example, the sequence of mRNA, encoding lactoferrin in seminal vesicles was described (GenBank Acc No. AAN75578). From human bone marrow lactoferrin gene was cloned, which is a variant of alternative splicing (GenBank Acc No. AAR12276). Also published a number of nucleotide sequences of human lactoferrin gene from mammary gland and their corresponding amino acid sequences, which have some differences on specific amino acid residues (see, for example, (GenBank Acc Nos. AAA59511, AAB60324, AAG48753). In addition, described a number of polymorphisms, such as point-nucleotide polymorphism in exon 1 human lactoferrin gene, leading to a Lys/Arg polymorphism at position 29 of the N-terminal region of lactoferrin (see Velliyagounder K., et al. Infect. Immun., 2003, 71(11):6141-6147). Thus, useful within the framework of present invention is a human lactoferrin, which has at least 80%, more preferably at least 90%, more preferably at least 95%, and most preferably at least 99% amino sequence homology with the sequence of human lactoferrin, provided, for example, the sequence deposited in GenBank under access code Acc. No. AAA59511.

Taking into account the fact that it is impossible to obtain natural human lactoferrin in industrial scale, recombinant human lactoferrin is useful for implementing this invention. The sequence of human lactoferrin gene is known, for instance, from the international publication of application WO 92/21752 (December 10, 1992), and also see deposited in GenBank sequences mentioned above. There have been described techniques of expression for recombinant human lactoferrin and plasmid structures, used for this purpose (e.g. patent US 6,277,817, August 21, 2001). Very attractive methods of obtaining recombinant human lactoferrin are the expression methods in plant cell culture (application US 2004040062) or in methylotrophic yeast Pichia pastoris (international publication of application WO 01/32895, May 10, 2001). It is also presupposed within the framework of this invention to use human lactoferrin, obtained from the milk of transgene animals, e.g. milk cows. There have been described a highly efficient method to get through the expression of human lactoferrin in the mammary gland of goats, which intradurally injected recombinant replication-defective adenovirus (Han Z.S., et al. High-level expression of human lactoferrin in the milk of goats by using replication-defective adenoviral vectors. Protein Expr. Purif. (2007), v.53(1), p.225-231).

The main significant difference between the declared preparation and all the earlier known pharmaceuticals, containing lactoferrin, is in the fact that due to a high purification degree of the lactoferrin, included in the preparation composition, a maximum concentration of one isoform (with apparent molecular weight about 80 kDaltons) of apolactoferrin, the most active antiviral agent, is achieved, thus, guaranteeing its reliability and safety of its usage in therapeutic process.

The declared concentration of lactoferrin in the preparation allows to preserve lactoferrin for a long time in its native state, that is in the state of maximum physiologic activity.

The examples, given further, disclose the most preferable ways of implementing this invention and they are given only in order to explain its essence better. Any specialist in this area will understand that it is possible to have a lot of modifications, both regarding the materials used and the methods used without deviating beyond the framework of the invention.

### Example 1. - Comparative analysis of different human lactoferrin preparations.

Each sample was assessed according to the following parameters:
1) Content of human lactoferrin protein.
2) Degree of lactoferrin saturation with iron
3) Lactoferrin ability to bind iron
4) Human lactoferrin purity was assessed with the aid of denaturant electrophoresis in polyacrylamide gel in the presence of sodium dodecyl sulphate (SDS).
5) Human lactoferrin purity was assessed with the aid of high-performance liquid chromatography (HLPC).

**Samples:**

| Sample Nos. | Preparation |
|---|---|
| 1-3 | Commercial Laprot preparation of lyopholized human lactoferrin |
| 4-13 | Human apolactoferrin with concentration of 20 mg/ml |

### Lactoferrin protein content in the samples

The "Laprot" preparation was obtained from P.A. Gertsen Moscow Research Oncology Institute. Lactoferrin protein content in Laprot preparation (Samples 1-3) varied in the range between 2.7 and 3.3 mg per 10 mg of the lyophilizated preparation.

Apolactoferrin protein (Samples 4-13) was obtained from the serum of human milk by salting out with ammonium sulfate and cation-exchange chromatography in accordance with previously described method (USSR Patent No.1709606 01.03.1990) with some modifications. Cation-exchange chromatography was performed on a column of 25 mm diameter with a volume of 50 ml of cation exchanger. As the cation exchanger Toyoperl CM-650S (TosoHaas Part#43253) was used. Flow rate 30 ml/hour, peristaltic pump Microperpex (Pharmacia). Protein was detected in eluate by means ofUvicord SII (Pharmacia) detector, fractions were collected by means of Multirak (Pharmacia) fraction collector.

Determination of the concentration of lactoferrin in solutions was carried out using a set of reagents for immunoenzyme determination of lactoferrin in the serum "Lactoferrin-ELISA-Best" D-4156 (ZAO "Vector-Best") according to the instruction, attached to the set. Counting of results was carried out with Excel program, using the polynomial approximation of the second degree.

In addition, the concentration of lactoferrin was determined spectrophotometrically with Ultrospec 11 spectrophotometer in a cell with optical path length of 1 cm. The concentration of lactoferrin in the preparation was determined by the formula: Concentration (mg/ml)=A₂₈₍₎/1.27, as described in EP 0 253 395 B2.

Apolactoferrin protein content in Samples 4-13 varied in the range between 15.2 and 20.0 mg per 1 ml of the solution.

### Degree of human lactoferrin saturation with iron

The degree of human lactoferrin saturation with iron was measured by spectrophotometric method by the ratio A₄₆₅/A₂₈₀. Table 1 presents the degree of saturation with iron (%) for all the tested samples.

**Table 1. Degree of human lactoferrin saturation with iron for various samples.**

| Sample | % of iron saturation |
|---|---|
| 1 | 25 |
| 2 | 32 |
| 3 | 39 |
| 4 | 3.7 |
| 5 | 3.6 |
| 6 | 3.4 |
| 7 | 3.9 |
| 8 | 5.1 |
| 9 | 3.5 |
| 10 | 3.9 |
| 11 | 4.2 |
| 12 | 4.6 |
| 13 | 3.5 |

Thus, for the commercial preparation Laprot (Samples 1-3), the degree of lactoferrin saturation with iron varied within the wide range between 25 and 39%. This level of saturation with iron corresponds to the average saturation level of natural human lactoferrin.

In contrast, the apolactoferrin preparation showed that the degree of iron saturation varied between 3.4 and 5.1 %.

### Human lactoferrin affinity for iron

When adding iron ions as a concentrated solution of ammonium sulfate-iron, it was stated that all the samples, containing human lactoferrin in various quantities, demonstrated 100% of lactoferrin saturation with iron ions.

### Assessment of purity for human lactoferrin preparations by the technique of denaturant electrophoresis in polyacrylamide gel (PAGE)

Analysis of the samples was carried out by PAGE in the presence of sodium dodecyl sulfate (SDS) in the system, described by Laemmli U.K. (Cleavage of the structural proteins during the assembly of the head of the bacteriophage T4. Nature (1970), v.227, p.680-685). In order to perform the test, 20 µg of human lactoferrin, samples Nos. 1-3 (Laprot) and 10 µg of human lactoferrin, samples Nos. 4-13, were applied to the paths.

Large amounts of human lactoferrin, used for carrying out the test, were necessary in order to evaluate the probable decomposition products of human lactoferrin, mainly for the lyophilizated samples that demonstrated much lower human lactoferrin concentration than was supposed.

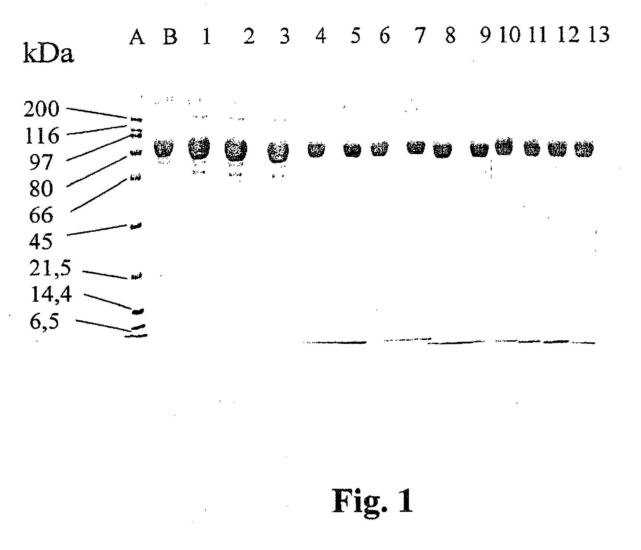

The obtained information is presented in Fig. 1. All the samples had a clearly marked major band, corresponding to apparent molecular weight of 80,000 Daltons. In particular, the presence of protein with the molecular weight of 80,000 Daltons was indicated for all samples No.4-13, together with insignificant amount of other proteins, likely to be decomposition products of human lactoferrin.

On the other hand, samples No.1-3 (commercially available preparation Laprot) demonstrated one major band, corresponding to apparent molecular weight of 80,000 Daltons and the presence of two more minor bands, corresponding to 75,000 and 70,000 Daltons. One of these two bands was also present in lactoferrin, used as reference specimen.

### Assessment of purity for the human lactoferrin preparations bv high performance liquid chromatography method (HLPC)

In order to determine purity of proteins and to detect possible admixture, 50 µg of human lactoferrin from each sample was tested using high performance liquid chromatography.

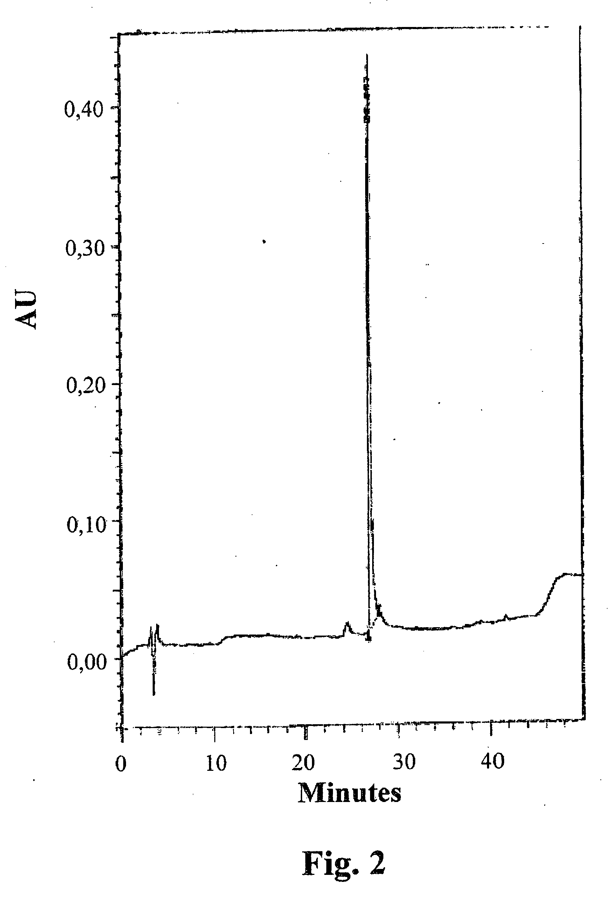

The chromatograms were identical for all samples No.1-3. A typical chromatogram, obtained for sample No.1, is shown in Fig. 2.

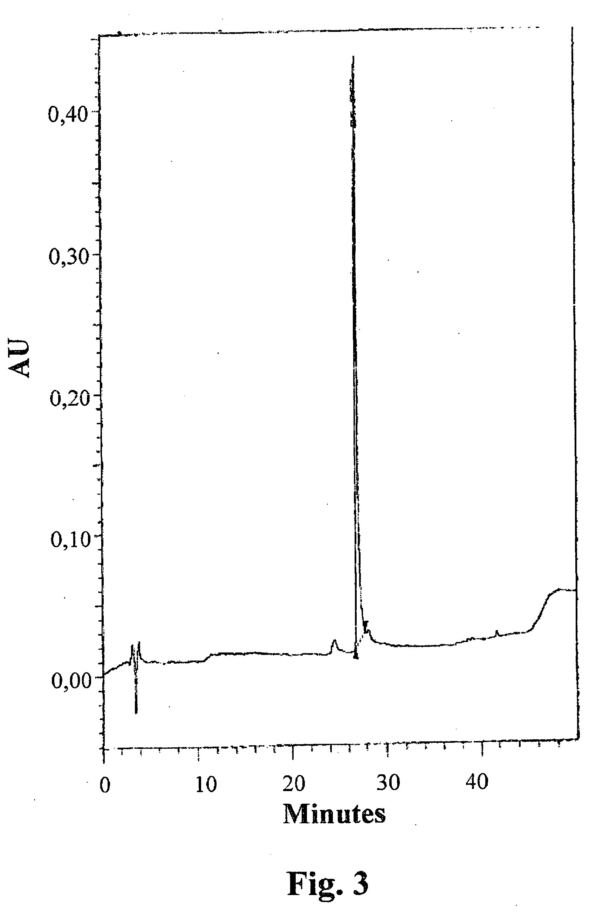

The chromatograms were identical for all samples No.4-13, in the form of solution. A typical chromatogram, obtained for sample No.13, is shown in Fig. 3.

The high performance liquid chromatography results for Samples 1-13 showed that decomposition products of human lactoferrin were not present in any of the samples.

### Example 2. Clinical experience in intravenous administration of human lactoferrin.

The human lactoferrin preparation was administered either as monotherapy or combined with interferon, prolonged interferon, ribavirin and cytostatics in combination with extracorporal methods of detoxification.

### Case study 1

Patient G.A.L., 30 years old man. Diagnosed with combined hepatitis B+C. According to polymerase chain reaction, initial viremia is high (>100,000 IU/ml). The disease duration constituted 5 years, according to the documented data. However, judging by the case history data, one can assume that the actual duration of the disease was about 14 years.

The patient was treated in an in-patient clinic. There were attempts to administer pulse doses of 500 mg of apolactoferrin against the background of treating with reaferon, 3 million units every other day. This method of administration did not yield any changes, either in the viremia level or in the biochemical indices. In 7-9 days after the start of the treatment aggravation of a catarrhal disease was observed. These catarrhal manifestations were obliterated and disappeared naturally, without interfering in the therapy process of the main disease.

Later the two-hour administration scheme was used, 20 mg every 2 hours for 10 hours. As a result of using this technique for two days successively, against the background of preliminary long-term treatment with reaferon, elimination of hepatitis C virus took place. Simultaneously, hepatitis B virus was not detected once, however, it was already detected again on the same level in a week. Hepatitis C virus was not detected either against the background of treatment with reaferon in the next three months or after the end of this treatment for the next 9, 18, 27 and 36 months, which was confirmed by control testing. Treatment of hepatitis B was done in accordance with the standard scheme and ended successfully only after one year.

### Case study 2

Patient K.V.A, 21 years old man. Diagnosed with hepatitis C with 1B virus genotype. According to polymerase chain reaction, initial viremia is high (>100,000 IU/ml). The disease duration constituted 3 years, according to the documented data. However, judging by the case history data, one can assume that the actual duration of the disease was twice as large.

The treatment was carried out according to a scheme with a weekly periodicity, at that reaferon was not administered during apolactoferrin administration and during the one-week interval between administering apolactoferrin reaferon was taken twice in the dose of 3 million units. In 7-9 days after the start of the treatment aggravation of a catarrhal disease was observed. These catarrhal manifestations were obliterated and disappeared naturally, without interfering in the therapy process of the main disease.

Virus elimination took place in the third week of the complex treatment (apolactoferrin, reaferon, HDF. In 6, 16, 23 and 30 month after completion of treatment the laboratory information about stable absence of the virus in this patient's blood was received.

### Case study 3

Patient Sh.A.A., 29 years old man. Diagnosed with hepatitis C, caused by genotype 2a virus, discovered by accident during preventive examination in titer 3×10⁴ IU/ml. The disease duration constituted 2 months, according to the documented data, however, history with clinical manifestations of hepatitis C was about 2 years.

The therapy was ambulant, the human lactoferrin apoprotein preparation was administered by a seven-day scheme in conjunction with reaferon and extracorporal methods of detoxification. Since the beginning of the treatment the biochemical disorders in the blood was not detected, bilirubin and liver enzymes remained within the normal limits.

The elimination of the virus was observed at the end of the third week of a comprehensive treatment. In control studies after 6, 12, 24 and 33 months, there were no virus in the blood.

### Case study 4

Patient S.L.G., 37 years old man. Diagnosed with hepatitis C, caused by genotype 1b virus, discovered during preventive examination in titer 428000 IU/ml. The disease duration constituted 1 year. No clinical signs of hepatitis. No complains. Biochemical tests were normal. The complex administration of human apolactoferrin in combination with reaferon, ribavirin, lamivudine and extracorporal methods of detoxification on the seven-day scheme was conducted in two phases. At the end of the third week of the first phase the decrease of HCV titer to 500 IU/ml was observed. This significantly increased above normal liver enzymes and bilirubin in the blood, there were signs of immunosuppression. Therefore, after a week's break of the treatment, was carried out the second phase of an integrated treatment of the same scheme with reduced doses of ribavirin and lamivudine. The elimination of the virus was observed by the end of the first week of the second phase of treatment. Control studies for the next 13 months do not indicate the presence of virus in the blood. Biochemical indicators were also stabilized.

### Case study 5

Patient S.S.B., 47 years old man. Diagnosed with hepatitis C, caused by genotype 2A virus. The viremia is high, 2.2x 10⁵ IU/ml. It was a tenfold increase in enzymes in blood chemistry with a moderate increase of bilirubin to the upper limit of normal. The disease duration constituted 0,5 years, according to the documented data. However, judging by the case history data, one can assume that the actual duration of the disease was much longer, about 3 years.

The therapy was ambulant. The human lactoferrin apoprotein preparation was administered daily in the dose of 40 mg for three days and then in the dose of 20 mg for four days. Then a one-week interval was made and a second course of apolactoferrin therapy followed, when 40 mg of the preparation was administered daily. This therapy scheme was chosen, based on the assumption that the preparation possesses immune suppressant action. Bearing in mind that the development cycle for immune cells constitutes about 7 days, it was decided to alternate seven-day cycles of administering the apolactoferrin preparation, without the use of interferons.

The monotherapy with apolactoferrin resulted in partial elimination of the virus: the viremia was reduced from high to medium, 3×10⁴ IU/ml. The monotherapy with human lactoferrin apoprotein in this case provided only a partial elimination of hepatitis C virus.

It comes to our attention that in the second and in the third week of treatment enzyme release increased while the mean level of viremia was preserved. It was noticed that in 7-9 days after the start of the treatment aggravation of a catarrhal disease was observed. These catarrhal manifestations were obliterated and disappeared naturally, without interfering in the therapy process of the main disease.

The next stage of the patient's treatment after using apolactoferrin monotherapy was complex treatment with interferon preparations. The course of treatment started with a pulse dose of 20 mg of apolactoferrin every 2 hours until reaching the dosage of 100 mg. Then the apolactoferrin preparation was administered daily per 40 mg without observing any weekly periodicity. Starting with the fifth day of apolactoferrin administration, Alfa 2A (Reaferon) interferon preparation was taken in the dose of 3 million units every other day. This decision was made because of the high initial level of AlAT (SGPT) and AsAT (SGOT) in order to implement hepatoprotection. Within 3 days after administering the two-hour scheme of apolactoferrin this patient demonstrated more than three times reduction of the AlAT (SGPT) and AsAT (SGOT) levels while preserving average viremia. The bilirubin level reduced considerably, testifying to a marked hepatoprotective action of the two-hour scheme. In a week after reaferon administration was started, the virus elimination took place, then apolactoferrin administration stopped and reaferon treatment continued in the same amount. Further on, two more negative PCR tests were obtained within two weeks and then reaferon administration was stopped.

In a month after the end of the treatment recurrence took place. Viremia rose to the medium level, being accompanied by high enzyme release. By the start of the treatment viremia was 5×10⁶ IU/ml, enzyme release was high, although it was twice less than the initial level. Then for two days successively the complex therapy with administering apolactoferrin according to the two-hour scheme was used, and then it was taken daily in the dose of 20 mg, while observing weekly periodicity in order to prevent possible immunity of the virus to the preparation. Reaferon was replaced with Intron A in the dose of 5 million units every other day, then the treatment was complemented by ribavirin, 1,200 mg daily.

It was decided to start this stage of treatment with administering Intron, assuming that apolactoferrin "marks" the virus by binding with its epitope E2, thus, making it visible for the immune system and impeding its penetration into the hepatocyte. Then, for two days after the first Intron injection the two-hour scheme of apolactoferrin administration was used, 20 mg every two hours until the dosage of 100 mg was reached, then apolactoferrin administration was continued in the dose of 20 mg daily.

According to the test results, after two days of administering apolactoferrin, viremia level reduction from 5,000,000 to 400,000 IU/ml was detected together with a double reduction of enzyme level. A low level of antiviral IgM attracted attention as the one that did not suit the process intensity. After the beginning of treatment its level slightly increased, although it is hard to say whether it was connected with Introne or apolactoferrin action. By the end of the first week of complex treatment the viremia level reduced to low and by the end of the second week of treatment, without apolactoferrin administration, it dropped to minimum (630 IU/ml with the sensitivity threshold of 600 IU/ml). All the changes of the viremia level correlated with lowering the level of enzyme release, first of all AlAT (SGPT) and AsAT (SGOT). By the end of the third week of treatment the virus was not detected in blood and the biochemical tests were within the normal range. The patient was transferred to anti-recurrence treatment with Pegasis preparation in the dose of 135 mg weekly. Dynamic observation for 4, 13 and 24 months. No recurrence.

### Case study 6

Patient A.A.N., 37 years old woman. Diagnosed with hepatitis C with 1B virus genotype. The viremia is high, 1000.000 IU/ml. The disease duration constituted 2 years, according to the documented data.

The therapy was ambulant. The human lactoferrin apoprotein preparation was administered daily in the dose of 40 mg for three days and then in the dose of 20 mg for four days. Then a one-week interval was made and a second course of apolactoferrin therapy followed, when 40 mg of the preparation were administered daily. This therapy scheme was chosen, based on the assumption that the preparation possesses immune suppressant action. Bearing in mind that the development cycle for immune cells constitutes about 7 days, it was decided to alternate seven-day cycles of administering the apolactoferrin preparation.

The monotherapy with HL resulted in partial elimination of the virus: viremia was reduced from high to low 2×10³ IU/ml.

An increase of enzyme level was noticed, although all the values are within the normal range. In 7-9 days after the start of the treatment aggravation of a catarrhal disease was observed. These catarrhal manifestations were obliterated and disappeared naturally, without interfering in the therapy process of the main disease.

The next stage of the patient's treatment after partial success of using apolactoferrin monotherapy was complex treatment with interferon preparations. The course of treatment started with a pulse dose of 20 mg of apolactoferrin every 2 hours until reaching the dosage of 100 mg. Then the apolactoferrin preparation was administered daily in the dose of 40 mg without observing any weekly periodicity. Together with apolactoferrin administration, Alfa 2A (Reaferon) interferon preparation was taken in the dose of 3 million units every other day.

The first administration of reaferon preceded the two-hour scheme of apolactoferrin administration. The virus elimination took place five days after the start of treatment with apolactoferrin. Reaferon was administered in the same dose of 3 million units every other day. It is important to emphasize that the virus elimination was accompanied by a relative rise of AlAT (SGPT) and AsAT (SGOT) levels, although they stayed within the normal range. Viremia remained on the medium level before and after administering the two-hour scheme. Taking into account the 1B virus genotype, apolactoferrin administration was prolonged up to 8 days and the reaferon therapy continued in the same volume. However, one week after apolactoferrin administration stopped, viremia increased to the medium level. Another course of apolactoferrin administration started in the dose of 20 mg daily, but the control test on the fourth day of the treatment revealed high viremia. A two-week break was made in administering apolactoferrin.

The treatment course was complemented by ribavirin in the dose of 1,000 mg daily, reaferon was replaced with interferon alfa-2B (Intron) in the dose of 5 million units every other day. Apolactoferrin administration started with the approved two-hour scheme and then continued in the dose of 20 mg daily without observing weekly periodicity. At the time of starting the treatment viremia was medium. The amount of antiviral immunoglobulin M was significant. Intron was administered, starting with the 5^{th} day of apolactoferrin intake. By the sixth day of treatment, i.e. by the start of Intron administration, the patient demonstrated viremia increase up to 430 thousand IU/ml, correlating with biochemical indices in blood tests. A high level of antiviral IgM remained. After the start of Intron administration there was a quick reduction of viremia level within five days and then the virus was eliminated within two weeks. After that apolactoferrin administration stopped and Intron administration continued in the same range. The control tests, done 5, 12, 19 and 25 months after the end of apolactoferrin administration, did not reveal viremia.

### Case study 7

Patient M.T.I., 47 years old woman. Diagnosed with hepatitis C, caused by genotype 1b virus. The long history of liver pathology about 6 years, chronic pancreatitis. The HCV diagnosis was laboratory-discovered during preventive examination. The disease duration constituted 1,5 years, according to the documented data. Initial viremia 1×10⁷ IU/ml.

The patient was treated in an in-patient clinic. Baseline biochemical parameters of AlAT (SGPT), AsAT (SGOT) within normal limits, bilirubin increased twice, 10 times increased ALP.

The comprehensive treatment was in the seven-day mode, using a two-hour scheme used native lactoferrin (preparation Laprot), reaferon, lamivudine, ribavirin and extracorporal methods of detoxification. As a single dose 50 mg of lyophilized Laprot preparation was used. By the end of the third week of complex treatment the increase of liver enzymes observed of almost 2-fold and reduced ALP to normal. Viremia decreased to 6.×10⁵ IU/ml. After a two-week break, the second phase of integrated therapy with the Laprot preparation conducted. Throughout the second phase of viremia remained within 1.2×10⁵-2.3×10⁵ IU/ml. At the end of 3 weeks 2 stages declined viremia below 500 IU/ml was noted. Given the signs of immunosuppression, it was a two-week break of complex therapy. During those two weeks only prolonged interferon Pegasys was used, used 1 time per week. The control study revealed preservation of viremia at the level of 600 IU/ml.

At the beginning of the third phase of treatment under the scheme viremia increased to 3 IU/ml and remained at that level until the end of the third phase. The patient was transferred to the scheme of long-term use of prolonged interferon. Control studies after 6, 12 and 20 months show the presence of viral load within 1 × 10³-1 × 10⁴ IU/ml.

### Case study 8

Patient Z.A.A., 18 years old man. Diagnosed with hepatitis C, caused by genotype 1b virus. The disease duration constituted about 5 years. Initial viremia was 500000 IU/ml. In-patient treatment, comprehensive, with the use of the Laprot preparation in three stages. Each stage consisted of a three-week cycle in the seven-day mode with a two-hour scheme. Biochemical parameters for all three phases, conducted intermittently for two weeks, practically remained at the initial level within normal limits. By the end of the first phase of viremia declined to 500 IU/ml. By the beginning of the second phase of the virus in the blood was not detected. However, in the middle of the first seven days of the second phase, he again manifested itself in the 2.2×10⁴ IU/ml. The entire third phase of treatment, i.e. another 21 days was accompanied by persistent viremia indicators within 1×10³-1.6×10³ IU/ml. Given the age of the patient and significant immunosuppression at the end of 3 stages, it was decided to transfer the patient to a long course of prolonged interferon Pegasys. Control studies after 6, 13, 21 and 29 months show a conservation of viremia practically at baseline.

### Case study 9.

Patient N.A.V., 33 years old man. Diagnosed with hepatitis C, caused by genotype 1b virus. Duration of the disease of about 10 years was reliably documented. In case history were repeated interferon courses without positive effect. Initial viremia 2.1×10⁷ IU/ml.The course of combined therapy with the Laprot preparation on a two-hour scheme, seven days cycles carried out in two phases. By this time, declined initially increased in 1,5 times biochemical indices. The antiviral effect of the treatment received too little.

### Conclusion

These clinical observation are typical for the two groups of patients with the use of one group of native human lactoferrin contained in the Laprot preparation - 9 observations (one of them has been a patient's treatment refusal in the first week of treatment for an unknown reason), and in the other group the human apolactoferrin, obtained by the authors above described method - 51 observation (treatment refusal in one case, a patient with an unstable psyche because of the long history of drug abuse).

Apolactoferrin monotherapy was carried out in 5 patients, but because of the lack of antiviral effect became the new combined therapy with the use of interferons, cytostatic agents and methods of extracorporal detoxification.

Not intending to be limited by any scientific theories, the inventors suppose that the parenterally administered apolactoferrin is tropic for the hepatitis C virus and interacts with it, presenting it to the immune system and impeding its penetration into the hepatocyte. Long-term and regular administration of apolactoferrin leads to some immune suppressing effects, indirectly characterized by viremia growth in case of monotherapy and by the fact that all the patients develop catarrhal changes in about a week after the start of treatment. However, no allergic reactions were noticed in any cases.

The most vivid effect is achieved when apolactoferrin is administered according to the two-hour scheme, 5-6 times in the dose of 20 mg every two hours, together with preliminary treatment with interferon preparations. It is possible that a fractional way of administering the preparation is clinically expedient, when it is taken with a 2-3 hours interval for a longer period, i.e. several days or, possibly, weeks.

## Claims

1. A composition for use in the treatment of HCV infection by administration to a patient,
wherein said composition comprises human lactoferrin, said human lactoferrin has an iron content of not exceeding 6% and lactoferrin comprised in said composition has at least 80% amino sequence homology with the sequence of human lactoferrin, and
wherein said treatment further comprises administering interferon to the patient.

2. A composition comprising human lactoferrin and interferon for use in the treatment of HCV infection, wherein said human lactoferrin has an iron content of not exceeding 6% and wherein lactoferrin comprised in said composition has at least 80% amino sequence homology with the sequence of human lactoferrin.

3. The composition of claim 1 or 2 for use in the treatment of HCV infection according to claim 1 or 2, wherein said human lactoferrin has an apparent molecular weight of about 80 kDa.

4. The composition of claim 1 or 2 for use in the treatment of HCV infection according to claim 1 or 2, wherein said human lactoferrin is present in an amount of about 20 mg.

5. The composition of claim 1 or 2 for use in the treatment of HCV infection according to claim 1 or 2, wherein said HCV infection is a chronic HCV infection.

6. The composition of claim 1 or 2 for use in the treatment of HCV infection according to claim 1 or 2, wherein said HCV is the genotype 1b HCV.

7. A kit for use in the treatment of HCV infection comprising:
a first container having therein a therapeutically effective amount of human lactoferrin, wherein said human lactoferrin has an iron content of not exceeding 6%;
a second container having therein a therapeutically effective amount of interferon.

8. The kit of claim 7 for use in the treatment of HCV infection according to claim 7, wherein the lactoferrin comprised in said first container has at least 80% amino sequence homology with the sequence of human lactoferrin.

9. The kit of claim 7 for use in the treatment of HCV infection according to claim 7, wherein said human lactoferrin has an apparent molecular weight of about 80 kDa.

10. The kit of claim 7 for use in the treatment of HCV infection according to claim 7, wherein said human lactoferrin is present in an amount of about 20 mg.

11. The kit of claim 7 for use in the treatment of HCV infection according to claim 7, wherein said HCV infection is a chronic HCV infection.

12. The kit of claim 7 for use in the treatment of HCV infection according to claim 7, wherein said HCV is the genotype 1b HCV.

## Patentansprüche

1. Zusammensetzung zur Anwendung in der Behandlung von HCV Infektion durch Verabreichung an einen Patienten,
wobei die Zusammensetzung humanes Lactoferrin umfasst, wobei das humane Lactoferrin einen Eisengehalt hat, welcher 6% nicht übersteigt, und das Lactoferrin, welches in der Zusammensetzung enthalten ist, mindestens 80% Aminosequenzhomologie mit der Sequenz von humanem Lactoferrin aufweist, und
wobei die Behandlung weiterhin die Verabreichung von Interferon an den Patienten umfasst.

2. Zusammensetzung umfassend humanes Lactoferrin und Interferon zur Anwendung in der Behandlung von HCV Infektion, wobei das humane Lactoferrin einen Eisengehalt hat, welcher 6% nicht übersteigt, und wobei das Lactoferrin, welches in der Zusammensetzung enthalten ist, mindestens 80% Aminosequenzhomologie mit der Sequenz von humanem Lactoferrin aufweist.

3. Zusammensetzung gemäß Anspruch 1 oder 2 zur Anwendung in der Behandlung von HCV Infektion gemäß Anspruch 1 oder 2, wobei das humane Lactoferrin ein scheinbares Molekulargewicht von ungefähr 80 kDa hat.

4. Zusammensetzung gemäß Anspruch 1 oder 2 zur Anwendung in der Behandlung von HCV Infektion gemäß Anspruch 1 oder 2, wobei das humane Lactoferrin in einer Menge von ungefähr 20 mg vorliegt.

5. Zusammensetzung gemäß Anspruch 1 oder 2 zur Anwendung in der Behandlung von HCV Infektion gemäß Anspruch 1 oder 2, wobei die HCV Infektion eine chronische HCV Infektion ist.

6. Zusammensetzung gemäß Anspruch 1 oder 2 zur Verwendung in der Behandlung von HCV Infektion gemäß Anspruch 1 oder 2, wobei das HCV Genotyp 1b hat.

7. Kit zur Anwendung in der Behandlung von HCV Infektion, umfassend:
ein erstes Behältnis, welches darin eine therapeutisch effektive Menge an humanem Lactoferrin hat, wobei das humane Lactoferrin einen Eisengehalt hat, welcher 6% nicht übersteigt;
ein zweites Behältnis, welches darin eine therapeutisch effektive Menge an Interferon hat.

8. Kit gemäß Anspruch 7 zur Anwendung in der Behandlung von HCV Infektion gemäß Anspruch 7, wobei das Lactoferrin, welches im ersten Behältnis enthalten ist, mindestens 80% Aminosequenzhomologie mit der Sequenz von humanem Lactoferrin aufweist.

9. Kit gemäß Anspruch 7 zur Anwendung in der Behandlung von HCV Infektion gemäß Anspruch 7, wobei das humane Lactoferrin ein scheinbares Molekulargewicht von ungefähr 80 kDa hat.

10. Kit gemäß Anspruch 7 zur Anwendung in der Behandlung von HCV Infektion gemäß Anspruch 7, wobei das humane Lactoferrin in einer Menge von ungefähr 20 mg vorliegt.

11. Kit gemäß Anspruch 7 zur Anwendung in der Behandlung von HCV Infektion gemäß Anspruch 7, wobei die HCV Infektion eine chronische HCV Infektion ist.

12. Kit gemäß Anspruch 7 zur Anwendung in der Behandlung von HCV Infektion gemäß Anspruch 7, wobei das HCV Genotyp 1b hat.

## Revendications

1. Composition destinée à être utilisée dans le traitement d'une infection par le HCV par l'administration à un patient,
où ladite composition comprend la lactoferrine humaine, ladite lactoferrine humaine a une teneur en fer ne dépassant pas 6% et la lactoferrine contenue dans ladite composition présente au moins 80% d'homologie de séquence d'acides aminés avec la séquence de lactoferrine humaine, et
où ledit traitement comprend en outre le fait d'administrer un interféron au patient.

2. Composition comprenant la lactoferrine humaine et l'interféron destinée à être utilisée dans le traitement d'une infection par le HCV, où ladite lactoferrine humaine a une teneur en fer ne dépassant pas 6% et où la lactoferrine contenue dans ladite composition présente au moins 80% d'homologie de séquence d'acides aminés avec la séquence de lactoferrine humaine.

3. Composition de la revendication 1 ou 2 destinée à être utilisée dans le traitement d'une infection par le HCV selon la revendication 1 ou 2, où ladite lactoferrine humaine a un poids moléculaire apparent d'environ 80 kDa.

4. Composition de la revendication 1 ou 2 destinée à être utilisée dans le traitement d'une infection par le HCV selon la revendication 1 ou 2, où ladite lactoferrine humaine est présente en une quantité d'environ 20 mg.

5. Composition de la revendication 1 ou 2 destinée à être utilisée dans le traitement d'une infection par le HCV selon la revendication 1 ou 2, où ladite infection par le HCV est une infection chronique par le HCV.

6. Composition de la revendication 1 ou 2 destinée à être utilisée dans le traitement d'une infection par le HCV selon la revendication 1 ou 2, où ledit HCV est le HCV de génotype 1b.

7. Kit destiné à être utilisé dans le traitement d'une infection par le HCV comprenant :
un premier récipient contenant dans celui-ci une quantité thérapeutiquement efficace de lactoferrine humaine, où ladite lactoferrine humaine a une teneur en fer ne dépassant pas 6% ;
un deuxième récipient contenant dans celui-ci une quantité thérapeutiquement efficace d'interféron.

8. Kit de la revendication 7 destiné à être utilisé dans le traitement d'une infection par le HCV selon la revendication 7, où la lactoferrine contenue dans ledit premier récipient présente au moins 80% d'homologie de séquence d'acides aminés avec la séquence de lactoferrine humaine.

9. Kit de la revendication 7 destiné à être utilisé dans le traitement d'une infection par le HCV selon la revendication 7, où ladite lactoferrine humaine a un poids moléculaire apparent d'environ 80 kDa.

10. Kit de la revendication 7 destiné à être utilisé dans le traitement d'une infection par le HCV selon la revendication 7, où ladite lactoferrine humaine est présente en une quantité d'environ 20 mg.

11. Kit de la revendication 7 destiné à être utilisé dans le traitement d'une infection par le HCV selon la revendication 7, où ladite infection par le HCV est une infection chronique par le HCV.

12. Kit de la revendication 7 destiné à être utilisé dans le traitement d'une infection par le HCV selon la revendication 7, où ledit HCV est le HCV de génotype 1b.
